# EUROPEAN PATENT APPLICATION

(11) **EP 3 778 035 A1**
(43) Date of publication of application: **17.02.2021**
(21) Application number: 19781755.4
(22) Date of filing: 02.04.2019
(51) Int. Cl.: B05B 17/06

(54) **MICROPOROUS ATOMIZING PLATE**

(30) Priority: 03.04.2018 CN 201810286104
(71) Applicant: Sunshine Lake Pharma Co., Ltd., Dongguan, Guangdong 523000 (CN)
(72) Inventor: CAO, Li, Dongguan, Guangdong 523871 (CN); ZHANG, Xuemei, Dongguan, Guangdong 523871 (CN); HU, Gang, Dongguan, Guangdong 523871 (CN); HUANG, Fangfang, Dongguan, Guangdong 523871 (CN)
(74) Representative: Hoffmann Eitle
(86) International application number: PCT/CN2019/080905
(87) International publication number: WO 2019/192441

(57) **Abstract**

A microporous atomizing plate, comprising a vibration slice (1) having a water immersion surface (6) and a backwater surface (7); the vibration slice (1) is provided with a microporous area (3), and the microporous area (3) is distributed with a plurality of atomizing holes (4): the backwater surface (7) of the vibration slice (1) has a surface roughness less than 150 nano. In this microporous atomizing plate, the surface roughness of the vibration slice is optimized, making the distribution of the atomizing holes in the vibration slicet more uniform, such that the particle size distribution of the sprayed droplets after atomization is narrower and more uniform, and the atomization effect of quantitative positioning dosing is realized.

## Description

### FIELD OF THE INVENTION

The invention relates to the field of liquid atomization devices, and especially relates to a microporous atomization sheet.

### BACKGROUND OF THE INVENTION

In the prior art, the microporous atomization sheet is mainly composed of a piezoelectric ceramic sheet and a vibration sheet attached to the ceramic sheet. One lead is respectively drawn from the piezoelectric ceramic sheet and the vibration sheet respectively, and a voltage is applied to the atomization sheet during use. When the excitation electric signal is transmitted to the piezoelectric ceramic sheet through the lead, the piezoelectric ceramic sheet is caused to vibrate, which further drives the vibration sheet to vibrate together. There are several micropores of uniform size distributed on the vibration sheet. Under the excitation of vibration, the liquid surface is continuously squeezed. After reaching a certain frequency and amplitude, part of the liquid in contact with the vibration sheet is squeezed out through the micropores to form tiny droplets, generating atomization.

In the field of spray therapy, the microporous atomization sheet is an important part of a portable atomizer, and its performance directly affects the effect of atomizing administration. However, a drawback of the existing microporous atomization sheet is that the particle size distribution of the droplets after the vibration atomization is too wide and not concentrated, so that the proportion of drugs reaching the lesion is reduced, and the proportion of drugs spreading to other non-lesion areas is greatly increased. The utilization rate is low, and the curative effect is reduced. If the inhaled drug is a drug with a large side effect such as hormones, too much drugs entering the non-lesion area lead to an increase in side effects and severe harm to the patients.

Based on the above, we need to design a microporous atomization sheet that can solve the above problems.

### SUMMARY OF THE INVENTION

In order to solve the problems existing in the prior art, the aim of the present invention is to provide a microporous atomization sheet with good atomization effect. By optimizing the surface roughness of the microporous atomization sheet, the surface of the metal vibration sheet is flatter, and the distribution of micropores processed is more uniform, which makes the particle size distribution of the droplets sprayed by the atomization narrower, thereby achieving the atomization effect of quantification and lung positioning administration.

To achieve this, the present invention adopts the following technical solutions:
a microporous atomization sheet comprises a vibration sheet having a water immersion surface and a backwater surface. The vibration sheet is provided with a microporous area, the microporous area is distributed with a plurality of atomizing pores, and the backwater surface of the vibration sheet has a surface roughness less than 150 nm.

Specifically, because the uniformity of the particle size distribution of the atomized particles is inseparable from the uniformity of the atomizing pores, and the surface roughness of the vibration sheet has an important influence on the uniformity of the atomizing pores, to ensure the distribution quality of the size of atomized particle after atomization, it is necessary to control the surface roughness of the metal vibration sheet in the atomization sheet within a certain range. The invention studies the surface roughness of the vibration sheet in the microporous atomization sheet. The surface roughness of the vibration sheet is reduced by reducing the surface roughness of the backwater surface of the vibration sheet. When the atomization pores are processed on the surface of the vibration sheet, the size of the atomization pores can be distributed more uniformly, so that the normal distribution of the size of atomized droplet after atomization is narrower, and spraying is more concentrated.

It is worth noting that the inventors found through research that there was no distinction between the water immersion surface and the backwater surface before processing the atomization pores on the vibration sheet, and the surface roughness of the two surfaces is the same. After the atomization pores are processed on the vibration sheet, the surface roughness of the vibration sheet will not change. After the atomization pores are processed on the vibration sheet, if the surface roughness of the backwater surface is changed, the uniformity of the distribution of the atomization pores will change, and the normal distribution of the particle size of the droplets will change after atomization. If only the surface roughness of the submerged surface is changed, and the surface roughness of the backwater surface is unchanged, the uniformity of the distribution of the atomization pores will not be affected, and the normal distribution of the particle size of the droplets will not be affected after atomization. At the same time, the inventors have found through a large number of studies that when the surface roughness of the backwater surface of the vibration sheet is greater than 150 nm, the particle size distribution of the atomized particles is broad. Although the particle diameter is mainly distributed within 1 to 10 µm, it is still distributed to a certain extent within the range of 25 to 350 µm; when the surface roughness of the backwater surface of the vibration sheet is 150 nm, 100 nm, 50 nm, 10 nm (when the error does not exceed ± 20%), the diameter distribution of the particles atomized by using an atomizer including the microporous atomization sheet of the present invention is normal distribution, and the atomized particles are uniform and highly concentrated. The proportion of atomized particles with a particle diameter in the range of 1 to 10 µm is as high as 96% or more, which meets the needs of pulmonary administration. Thereby, the utilization rate of the drug can be greatly improved, and the dosage can be reduced. However, the smaller the surface roughness, that is, the smoother the surface of the vibration sheet, the higher the requirements for processing equipment and processing technology conditions, and the larger the corresponding cost. The inventors think that a microporous atomization sheet with suitable surface roughness can be chosen according the need and the balance between the atomization effect and material cost. In some embodiment, the surface roughness of the immersion surface of the vibration sheet is the same as that of the backwater surface. In other embodiment, the surface roughness of the immersion surface of the vibration sheet is different from that of the backwater surface.

Preferably, the material of the vibration sheet is metal or polymer material.

As a preferred technical solution, the surface roughness of the backwater surface of the vibration sheet is 10 to 120 nm.

As a preferred technical solution, the surface roughness of the backwater surface of the vibration sheet is 20 to 110 nm.

As a preferred technical solution, the surface roughness of the backwater surface is of the vibration sheet 50 to 100 nm.

As a preferred technical solution, the shape of the atomization pore is a bell mouth shape, and two ends of the atomization pore are a water inlet end and a water outlet end respectively, and a diameter of the water inlet end is larger than a diameter of the water outlet end.

Specifically, the water inlet end is located on the water immersion surface side, and the water outlet end is located on the backwater surface side. The use of the microporous atomization sheet with the bell mouth shape helps the liquid to enter the atomization pores to complete the atomization, and obtains ultrafine atomized particles.

As a preferred technical solution, the microporous area is provided with a plurality of protrusions, and the atomization pores are located on the protrusions.

Specifically, the microporous area is provided with a plurality of protrusions, which can effectively increase the contact area between the atomization sheet and the liquid, meanwhile make the particles passing through the atomization pores spray in different directions and the atomization direction more divergent, thereby effectively reducing the mutual collision and fusion between the atomized particles, resulting in increased particle size. In addition, using this structure can also effectively improve the efficiency of energy conversion, reduce power loss and reduce heat generation.

As a preferred technical solution, an anticorrosive layer is provided on the water immersion surface and / or the backwater surface of the vibration sheet.

Specifically, by providing an anticorrosive layer on the water immersion or backwater surface of the vibration sheet, or by providing an anticorrosive layer on both the water immersion surface and the backwater surface of the vibration sheet, the vibration sheet can be isolated from the liquid and prevented from corrosion by the liquid, so that the vibration sheet can be protected, the corrosion resistance of the atomization sheet is improved and the service life is extended.

As a preferred technical solution, the anticorrosive layer is made of polymer glaze.

Specifically, polymer glaze has good acid and alkali resistance and good anticorrosive effect.

The beneficial effects of the present invention are: a microporous atomization sheet is provided. By optimizing the surface roughness of the vibration sheet, the distribution of the atomization pores in the vibration sheet is more uniform, so that the particle size distribution of the droplets sprayed by atomization is more uniform, thereby achieving the atomization effect of quantitative positioning administration.

### Description of the drawings

Figure 1 is schematic diagram of the structure of the present invention.
Figure 2 is a cross-sectional view of a microporous atomization sheet in Example 1 of the present invention.
Figure 3 is a cross-sectional view of a microporous atomization sheet in Example 2 of the present invention.
Figure 4 is a partially enlarged schematic diagram at A in Figure 3;
Figure 5 is size distribution of particles obtained by atomization of the microporous atomization sheet in Example 1.
Figure 6 is size distribution of particles obtained by atomization of the microporous atomization sheet in Example 3.
Figure 7 is size distribution of particles obtained by atomization of the microporous atomization sheet in Example 4.
Figure 8 is size distribution of particles obtained by atomization of the microporous atomization sheet in Example 5.

Wherein, vibration sheet 1, hollow annular piezoelectric ceramic sheet 2, microporous area 3, atomization pores 4, lead 5, water immersion surface 6, backwater surface 7.

### Examples

In order to make the structural features and achieved effects of the present invention be further understood and recognized, the preferred embodiments and drawings are used in conjunction with the detailed description, as follows:

### Example 1

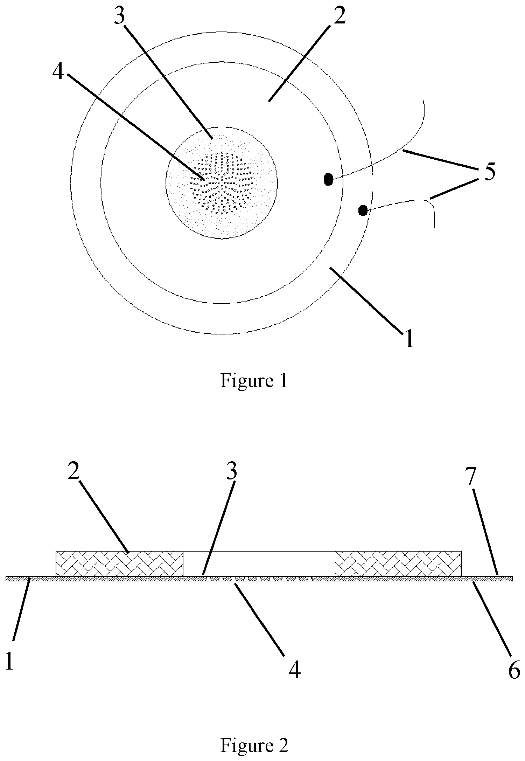

As shown in FIG. 1 and FIG. 2, a microporous atomization sheet includes a vibration sheet 1 and a hollow annular piezoelectric ceramic sheet 2 compounded in the vibration center of the vibration sheet 1. A lead 5 is respectively drawn from the vibration sheet 1 and the hollow annular piezoelectric ceramic sheet 2 for transmitting electrical signals.

A microporous area 3 is provided on the vibration sheet 1 corresponding to the center circular hole position of the hollow annular piezoelectric ceramic sheet 2, and a plurality of atomization pores 4 are distributed on the microporous area 3. The vibration sheet 1 has a water immersion surface 6 and a backwater surface 7. The shape of the atomization pore 4 is a bell mouth shape. The two ends of the atomization pore 4 are a water inlet end and a water outlet end respectively. The diameter of the water inlet end is greater than the diameter of the water outlet end. The diameter of the inlet end is 66 µm, and the diameter of the outlet end is 3 µm. The inlet end is connected to the water immersion surface 6, and the outlet end is connected to the backwater surface 7. The number of atomization pores 4 is 3000. The surface roughness of the backwater surface 7 of the vibration sheet 1 is 10 nm.

### Example 2

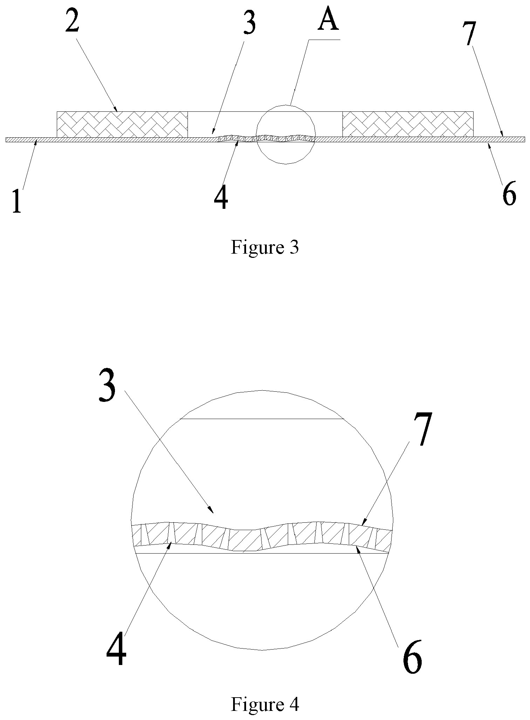

As shown in Figure 3 and Figure 4, the difference between Example 1 and this example is that: the microporous area 3 is provided with several protrusions, and the atomization pore 4 is located on the protrusion.

The other structures in this example are the same as those in Example 1, and will not be described herein.

### Example 3

The difference between Example 1 and this example is that: the surface roughness of the backwater surface 7 of the vibration sheet 1 is 50 nm.

The other structures in this example are the same as those in Example 1, and will not be described herein.

### Example 4

The difference between Example 1 and this example is that: the surface roughness of the backwater surface 7 of the vibration sheet 1 is 100 nm.

The other structures in this example are the same as those in Example 1, and will not be described herein.

### Example 5

The difference between Example 1 and this example is that: the surface roughness of the backwater surface 7 of the vibration sheet 1 is 150 nm.

The other structures in this example are the same as those in Example 1, and will not be described herein.

### Example 6

The difference between Example 1 and this example is that: an anticorrosive layer is provided on the water immersion surface 6 and / or the backwater surface 7 of the vibration sheet. The anticorrosive layer is made of polymer glaze.

The other structures in this example are the same as those in Example 1, and will not be described herein.

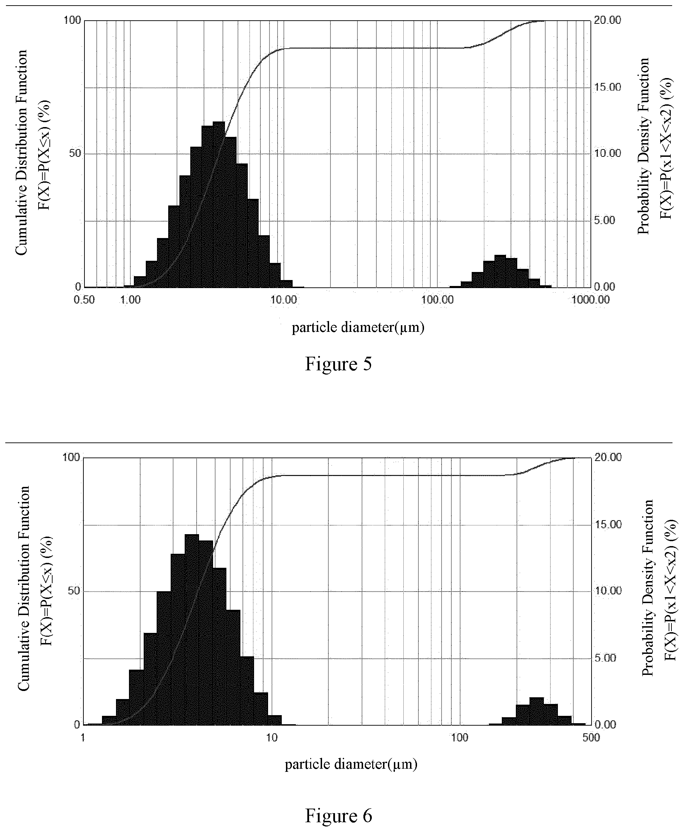

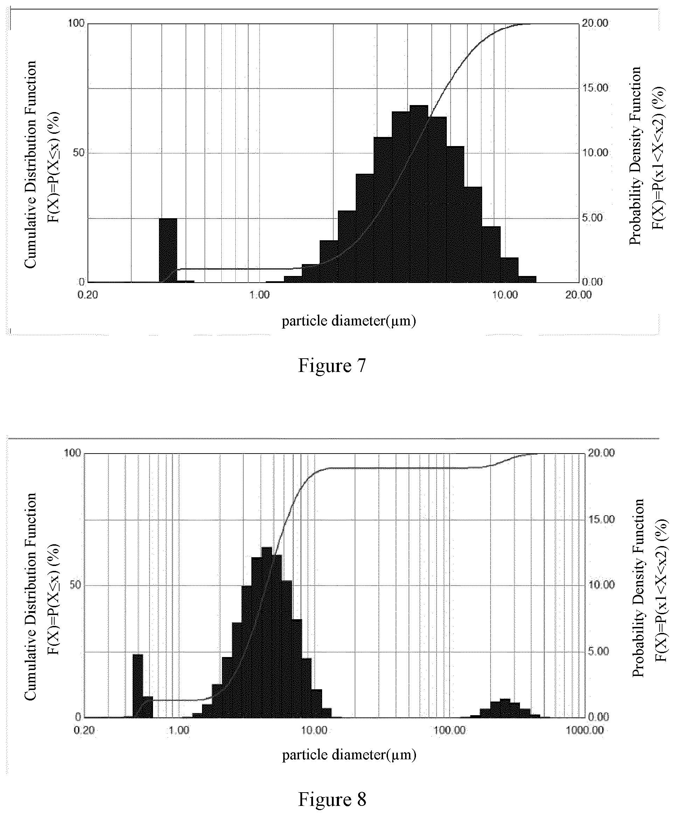

Testing of particle size distribution:
(1) testing method: the microporous atomization sheet of each of the above examples is separately installed in an atomizer, and an ultrasonic frequency is provided to the microporous atomization sheet. After the atomization sheet receives an electrical signal, a hollow annular piezoelectric ceramic sheet 2 mechanically vibrates. The mechanical vibration of the hollow annular piezoelectric ceramic sheet 2 drives the vibration sheet 1 to vibrate. The liquid in contact with the vibration sheet 1 is sprayed out through the atomization pore 4 during the vibration process to form atomized particles. The salbutamol solution is atomized by using the above atomizer, and the particle size distribution of the atomized particles obtained after the atomization is tested using the particle size analyzer.
testing results: Figure 5 shows the particle size distribution of the atomized particles obtained by the microporous atomization sheet in Example 1; Figure 6 shows the particle size distribution of the atomized particles obtained by the microporous atomization sheet in Example 3; Figure 7 shows the particle size distribution of the atomized particles obtained by the microporous atomization sheet in Example 4; Figure 8 shows the particle size distribution of the atomized particles obtained by the microporous atomization sheet in Example 5. Figures 5-8 show that when the surface roughness of the backwater surface 7 of the vibration sheet 1 is less than 150 nm, the particle size distribution of the atomized particles obtained by the microporous atomization sheet in above examples is concentrated, more than 96% are concentrated in the range of 1 to 10µm. It can be known that the use of the microporous atomization sheet of the present invention can make the normal distribution of the particle size of the atomized droplets narrower and more uniform, thereby achieving the atomization effect of quantitative positioning administration.

The parts that are not involved in the present invention are the same as the existing technology or can be implemented by using the existing technology, and are not repeated herein.

Finally, it should be noted that the above embodiments are only used to illustrate the technical solution of the present invention, and not to limit it; although the present invention has been described in detail with reference to the foregoing embodiments, those skilled in the art should understand that they can still modify the technical solutions described in the foregoing embodiments or equivalently replace some of the technical features; and these modifications or replacements do not depart from the spirit and scope of the technical solutions of the embodiments of the present invention.

## Claims

1. A microporous atomization sheet, comprising a vibration sheet (1) having a water immersion surface (6) and a backwater surface (7), wherein the vibration sheet (1) is provided with a microporous area (3), the microporous area (3) is distributed with a plurality of atomization pores (4), and the backwater surface (7) of the vibration sheet (1) has a surface roughness less than 150 nm.

2. The microporous atomization sheet of claim 1, the backwater surface (7) of the vibration sheet (1) has a surface roughness of 10 to 120 nm.

3. The microporous atomization sheet of claim 1 or claim 2, the backwater surface (7) of the vibration sheet (1) has a surface roughness of 20 to 110 nm.

4. The microporous atomization sheet of claim 1 or claim 2 or claim 3, the backwater surface (7) of the vibration sheet (1) has a surface roughness of 50 to 100 nm.

5. The microporous atomization sheet of any one of claims 1 to 4, the atomization pore (4) has a bell mouth shape, and two ends of the atomizing pore (4) are a water inlet end and a water outlet end respectively, and the diameter of the water inlet end is larger than the diameter of the water outlet.

6. The microporous atomization sheet of any one of claims 1 to 5, the microporous area (3) is provided with a plurality of protrusions, and the atomization pores (4) are located on the protrusions.

7. The microporous atomization sheet of any one of claims 1 to 6, the water immersion surface (6) and / or backwater surface (7) of the vibration sheet (1) is provided with an anticorrosive layer.

8. The microporous atomization sheet of any one of claims 1 to 7, wherein the anticorrosive layer is polymer glaze.
